# EUROPEAN PATENT SPECIFICATION

(11) **EP 0 500 938 B1**
(45) Date of publication and mention of the grant of the patent: **02.01.1997**
(21) Application number: 91910376.2
(22) Date of filing: 30.05.1991
(51) Int. Cl.: G01N 30/00, G01N 30/06, G01N 33/00, B01D 15/08, G01N 30/04

(54) **METHOD AND DEVICE FOR PREPARATION OF STANDARD VAPOUR-GAS MIXTURE OF SUBSTANCE TO BE ANALYZED**
METHODE UND GERÄT ZUR BEREITSTELLUNG EINES STANDARD-DAMPF-GAS-GEMISCHES EINER ZU UNTERSUCHENDEN SUBSTANZ
PROCEDE ET DISPOSITIF DE PREPARATION DE MELANGE DE VAPEUR-GAZ STANDARD DE SUBSTANCE A ANALYSER

(30) Priority: 06.08.1990 SU 4857234
(43) Date of publication of application: 02.09.1992
(73) Proprietor: INSTITUT NEORGANICHESKOI KHIMII SIBIRSKOGO OTDELENIA ROSSIISKOI AKADEMII NAUK, Novosobirsk, 630090 (RU); BANKOUL, Nikolai Viktorovich, CH-5425 Unterehrendingen (CH)
(72) Inventor: NADOLINNY, Vladimir Akimovich, Novosibirsk, 630090 (SU); KOVRIGIN, Viktor Mikhailovich, Novosibirsk, 630117 (SU); MAKOTCHENKO, Viktor Gerasimovich, Novosibirsk, 630117 (SU); SOLDATOV, Vladimir Prokopievich, Novosibirsk, 630055 (SU); NAZAROV, Albert Semenovich, Novosibirsk, 630090 (SU); MORALEV, Vadim Mikhailovich, Novosibirsk, 630072 (SU); YAKOVLEV, Ivan Ipatievich, Novosibirsk, 630072 (SU); KOVALEV, Dmitry Petrovich, Novosibirsk, 630055 (SU); BOLOBOLOV, Ustin Petrovich, Novosibirsk, 630117 (SU)
(74) Representative: Godwin, Edgar James
(86) International application number: SU9100109
(87) International publication number: WO9202812

(56) References cited:
- EP-A- 0 047 860
- EP-A- 0 360 901
- WO-A-90/04174
- US-A- 4 083 885
- US-A- 4 865 996
- PATENT ABSTRACTS OF JAPAN vol. 10, no. 64 (P-436)(2121) 14 February 1986 & JP-A-60205246
- ANALYTICAL CHEMISTRY vol. 56, no. 13, October 1984, COLUMBUS US pages 2500 - 2503 IOFFE, B.V. ET AL.
- Zhurnal neorganicheskoi khimii, Tom 27, Vyp. 8, 1982, Nauka, (Moscow), NADOLINNY V.A. et al., "Issledovanie soedinenia vuedrenia tetraoxida diazota v khloridftorid grafita metodom EPR" page 1981.
- Journal of Chromatography, Tom 300, 1984, Elsevier Science Publishers B.V. (Amsterdam) NAMIESNIK J., "Generation of standard gaseous mixtures", pages 90- 91.
- Zhurnal neorganicheskoi Khimii, Tom 23, No 6, 1978, NAZAROV A.S. et al., "Vzaimodeistvie grafita s rastvorami ftorida tsezia v triftoride khlora", page 1680.
- Zhurnal neorganicheskoi khimii, Tom 32, Vyp. 9, 1987, Nauka, (Moscow), Nadolinny V.A. et al., "Issledovanie interkalirovannykh soedineny ftorirovannogo grafita metodom EPR", page 2128

## Description

The present invention relates to methods of analysis of materials and determination of physico-chemical properties, and to devices for carrying out thereof, and more specifically it relates to methods and apparatuses for the preparation of vapour-gaseous mixtures of substance to be analysed.

There is known a method for the preparation of standard vapour-gaseous mixtures of substance to be analysed comprising thermostatting of an analysed substance and diffusion of the analysed substance into a free space blown-through by carrier gas ("Journal of Chromatography", v.300, 1984, Namesnik J. "Generation of standard gaseous mixtures", p.p.79-100).

There is also known a device for the preparation of standard vapour-gaseous mixtures of substance to be analysed carrying out said method and comprising a thermos tatting device accommodating a vessel for the analysed substance, and a means providing the penetration of the analysed substance into the free space blown-through by carrier gas ("Journal of Chromatography", v. 300, 1984, Namesnik J. "Generation of standard gaseous mixtures", p.92).

In said method the diffusion of the analysed substance into the carrier gas is carried out through means for the penetration of the analysed substance into the free space blown-through by the carrier gas from liquid or gaseous phase. The employment in said method of a great quantity of the analysed substance in liquid or gaseous state represents considerable ecological hazard because of continuous diffusion of said substance through the means for penetration, and in case of its damage.

In said method the analysed substance, before it enters the carrier gas in the form of gaseous phase, takes part in a number of processes, and passes through various states of aggregation. Those are as follows: the evaporation of the analysed substance from liquid state into the free space blown-through by a carrier gas, and the establishment of saturated vapour pressure over said substance, the dissolution of the analysed substance in the means for penetration, the diffusion through said means for penetration, and evaporation from the surface of the means for penetration. Each of the above mentioned processes is characterized by various rate depending on the number of parameters, and limits the time required to attain the equilibrium state of the device.

The combination of a great number of said processes wherein an analysed substance takes part related to said method for the preparation of standard vapour-gaseous mixtures does not allow to generate reproducibly vapour-gaseous mixtures of the analysed substance under the same conditions which results in low accuracy of said method.

In said device for carrying out of said method the means for the penetration of the analysed substance into the free space blown-through by the carrier gas is made in the form of a polymeric membrane, and the vessel for the analysed substance is represented by a test glass.

When carrying into effect the above mentioned method of preparation of standard vapour-gaseous mixtures for each specific analysed substance there exists a problem of selection of polymer for a membrane acting as the means for penetration, and as a result said device is not universal as regards substances to be analysed.

The operation of said device shows that since the initial moment of its thermostatting and blowing-through by the carrier gas a period of time from one to ten weeks is required to produce standard vapour-gaseous mixtures in a stream. It corresponds to the time required to establish steady rates of processes wherein the analysed substance participates. Theoretically it is possible to reduce this time at the expence of reducing of the membrane's thickness but practically it is unreal due to nonuniformities in the structure of polymers leading to mechanical damage thereof.

Besides, said device embodying the above mentioned method of preparation of standard vapour-gaseous mixtures operates unstably during prolonged operation or storing because of variation in physico-chemical properties of the polymeric membrane. As a consequence the mechanical damage of the membrane is possible which represents ecological hazard if toxic substances are used as those to be analysed. Long time it takes the device to go into steady operating conditions causes the problem of utilization of vapour-gaseous mixtures if the analysed substance is toxic.

The device described hereinabove embodying the above mentioned method can be applied only in dynamic mode of operation, i.e. for the generation of vapour-gaseous mixtures of the analysed substance in a stream of the carrier gas, which limits its operating capabilities.

US-A-4 083 855 discloses a method for alkylating hydrocarbons which involves the steps of putting the latter in contact with the alkylating agent at a molar ratio of hydrocarbons to the alkylating reagent varying from 1:1 to 10:1 at temperatures of between 0°C and 150°C, a pressure in the range between atmospheric and 500 psig through catalysis with a catalyst consisting of graphite having 5 to 50 wt.-% of Lewis acid fluoride (BF₃ or PF₅) intercalated in the lattice.

The method also envisages that the so-called catalyst is additionally intercalated with 0.5 to 75 wt.-% of Bronsted acid selected from hydrofluoric, hydrochloric, fluorosulfuric, and trifluoromethane-sulfonic acids, with a molar ratio of Bronsted acid to Lewis acid fluoride from 1.1:1 to 50:1.

The known method is concerned with intercalation of gaseous Lewis acids: for using the produced intercalated compounds as catalysts of alkylating reaction of various compounds, and for storage of gaseous Lewis acids at atmospheric pressure and ambient temperatures.

The known method fails to create standard vapour-gaseous mixtures for substances being analysed, because it utilizes the known property of intercalated compounds of graphite with gaseous Lewis acids to get decomposed during phase transition within a temperature interval of 100 - 200°C with simultaneous separation of the entire amount of introduced Lewis acid.

WO 90/04174 discloses a device for calibration of gas sensors, which comprises silicone rubber with a certain amount of dissolved gas, hermetically sealed with metal foil during storage and placed in close proximity to the sensor during calibration, building up a certain concentration of gas in a confined space. The known device is intended for calibration of sensors (transducers) and fails to calibrate instruments operating in dynamic and static modes. It has only a limited service life, particularly one-time use, and is unsafe ecologically because of interaction of toxic and chemically-active substances with polymeric rubber.

The present invention is based on the problem of providing a method for the preparation of standard vapour-gaseous mixtures of substance to be analysed according to which the diffusion of the substance to be analysed into a free space blown-through by carrier gas is carried out in a way enabling to increase ecological safety of the method as a result of exclusion of employment of large quantities of liquid or gaseous substances to be analysed, which is of especial importance if toxic substances are used as those to be analysed, reduce the time required to place the device to equilibrium state as a result of reduction of the number of processes wherein the analysed substance participates while the vapour-gaseous mixture is being prepared, and improve the reproducibility of diffusion parameters and thus to increase the accuracy of preparation of vapour-gaseous mixtures as a result of the exclusion of physico-chemical interactions between the analysed substance and a means for penetration as well as to provide an apparatus for the preparation of standard vapour-gaseous mixtures of the substance to be analysed carrying out said method wherein the means for the penetration of the analysed substance into a free space blown-through by the carrier gas is made so that to enable to combine the functions of the means for penetration with a vessel for the analysed substance, thus ensuring ecological safety during prolonged operation and storing of the apparatus, to make the apparatus universal as regards substances to be analysed, to reduce the time required for the apparatus to gain steady operating conditions by decreasing the length of diffusional path of the analysed substance, and to extend operational capabilities of the apparatus by its employment both in dynamic and static modes of operation.

The present invention provides a method for the preparation of standard vapour-gaseous mixtures of a substance to be analysed, the method comprising thermostatting of the substance and diffusion of the substance into a free space through which a carrier gas flows, characterised in that the diffusion of the substance into the free space through which the carrier gas flows is carried out during thermostatting, at a temperature of up to 50°C, from a solid fluorographite matrix which is chemically inert to the substance to be analysed, the substance being located in interplanar spaces of the matrix and escaping from the matrix by diffusion.

Fluorographite is also referred to as "graphite fluoride". (See: Yasushi Kita et al., Chemical Composition and Crystal Structure of Graphite Fluoride, Journal of the American Chemical Society, 101:14 (4 July 1979), pp. 3832-3841.) It is inert to a wide range of substances.

The invention also provides apparatus for the preparation of standard vapour-gaseous mixtures of a substance to be analysed, comprising a thermostatting device which accommodates an enclosure defining a free space through which a carrier gas is to flow, the enclosure containing the substance to be analysed, characterised in that the enclosure contains a solid fluorographite matrix which is chemically inert to the substance to be analysed, the substance being located in interplanar spaces of the matrix and escaping from the matrix by diffusion at a thermostatting temperature of up to 50°C.

The solid fluorographite matrix may be in the form of powder or in the form of at least one pellet. It is preferred that, if the solid fluorographite matrix is made in the form of a plurality of pellets, the pellets are arranged in the case at a preset distance from each other. It is convenient for at least one passage to be provided in a pellet of the solid fluorographite matrix.

It is convenient for the case with the solid fluorographite matrix to be installed rotatably.

The construction of the apparatus for the preparation of standard vapour-gaseous mixtures of the analysed substance carrying out the method according to the invention enables one to make it universal for analysed substances, liquid, gaseous and solid states of aggregation.

The composition of functions of the means for penetration and of the vessel for the analysed substance in said device in the solid fluorographite matrix enables both the apparatus for the preparation of standard vapour-gaseous mixtures itself and the procedure of filling of the claimed apparatus with the analysed substance to be simplified.

The decrease in the length of the diffusional path of the analysed substance in the means for penetration as a result of application of the solid fluorographite matrix to hundreds of Angströms in the claimed method helps reduce the time required for the apparatus to gain the steady mode of operation, according to the invention, from several hundreds of hours to several hours.

The reduction of time required for the device embodying said method to gain the steady mode of operation, and greater capacity of the fluorographite matrix as related to the introduced analysed substance (up to 20 mass %) prolongs the apparatus service life to several years.

Moreover, the claimed apparatus embodying the method according to the invention is ecologically safe and can be used both in dynamic and static modes of operation.

### Brief Description of the Drawings

The invention will now be described with reference to specific embodiments thereof taken in conjunction with the accompanying drawings, in which:
Fig.1 is a representation of apparatus for the preparation of standard vapour-gaseous mixtures of the analysed substance carrying out the method according to the invention (longitudinal cross section);
Fig.2 is an alternative embodiment of apparatus for carrying out the method according to the invention (longitudinal cross section);
Fig.3 is a cross section taken along line III-III in Fig.2 (scaled up);
Fig.4 is an alternative embodiment of apparatus for carrying out the method according to the invention (longitudinal cross section);
Fig.5 is a cross section taken along line V-V in Fig.4 (scaled up);
Fig.6 is a cross section taken along line VI-VI in Fig.5;
Fig.7 is another alternative embodiment of apparatus for carrying out the method according to the invention (longitudinal cross section);
Fig.8 is an alternative embodiment of the apparatus in Fig.7 (longitudinal cross section);
Fig.9 also shows the apparatus in Fig.8 (longitudinal cross section);
Fig.10 also shows the apparatus in Fig.9 (transverse cross section);
Fig.11 is a plot of variation of the level of vapour-gaseous mixture performed by the apparatus in Fig.1.

### Best Methods for Carrying Out the Invention

A method for the preparation of standard vapour-gaseous mixtures of substance to be analysed, according to the invention, resides in that the analysed substance is thermostatted, and the diffusion of the analysed substance into a free space blown-through by carrier gas is carried out from solid phase - a solid fluorographite matrix with the analysed substance located into interplanar spaces, as it will be described in greater detail below during the description of the apparatus embodying said method according to the invention.

The fluorographite matrix is inert to a wide class of substances to be analysed as regards both state of aggregation and chemical properties. The fluorographite matrix possesses high capacity for the analysed substance - up to 20 mass %. The diffusion of the analysed substance from the fluorographite matrix is impaired by boundary areas of approximately 100 Å and is carried out along the layers of the fluorographite matrix according to Fick's law.

The standard vapour-gaseous mixture of the analysed substance into the free space is attained when the saturated vapour pressure of the analysed substance is provided in said space or by dilution of the flow of the analysed substance vaporous phase from the surface of the fluorographite matrix by the stream of the carrier gas blown through the free space.

In the claimed method for the preparation of standard vapour-gaseous mixtures of the analysed substance the solid fluorographite matrix with the analysed substance located into interplanar spaces thereof performs the functions of the means for the penetration of the analysed substance and of the vessel for the analysed substance.

The time required to provide standard vapour-gaseous mixture of the analysed substance in the free space corresponds to the time required to establish the saturated vapour pressure of the analysed substance in the free space, and is determined by the processes of diffusion of the analysed substance from the fluorographite matrix and processes of sorbtion-desorbtion of the analysed substance from the surface of the fluorographite matrix into the free space.

In case when standard vapour-gaseous mixture of the analysed substance is generated in a stream of the carrier gas blowing through the free space, the time in which the stationary level of the vapour-gaseous mixture of the analysed substance is established in the stream of the carrier gas depends on the speed of the carrier gas stream and is determined by the time in which the stationary speeds of diffusional flops of the analysed substance and of the analysed substance flow from the surface of the fluorographite matrix into the carrier gas are established.

There should be outlined some cases which may occur on the surface of the fluorographite matrix depending on the state of aggregation of the analysed substance.

In case the analysed substance is liquid, a film of liquid analysed substance is formed on the surface of the fluorographite matrix as a result of diffusion of the analysed substance out of said matrix. If the analysed substance is gas, then in conditions under which the standard vapour-gaseous mixture is generated, several layers of the absorbed analysed substance with different binding energy between them are generated on the surface of the fluorographite matrix as a result of diffusion from its body.

The above mentioned features of behaviour of molecules of the analysed substance on the surface of the fluorographite matrix depending on the state of aggregation of the analysed substance are to be considered in carrying out the claimed method for the preparation of standard vapour-gaseous mixtures of the analysed substance in the apparatus according to the invention.

Specific embodiments of the claimed apparatus for the preparation of standard vapour-gaseous mixtures of the analysed substance carrying out the method according to the invention will be described hereinbelow in greater detail.

Apparatus for the preparation of standard vapour-gaseous mixtures of the substance to be analysed, according to the invention, carrying out the claimed method, will be described with reference to its specific embodiments both in dynamic and static modes of operation.

The apparatus according to the invention in Pig.1 comprises a thermostatting device 1, wherein a housing 2 is installed, wherein, in turn, there is installed a means 3 for the penetration of the analysed substance into the free space blown-through by carrier gas. The thermostatting device 1, for example a thermostat, is shown schematically in the drawing since it is not an object of the present invention.

The means 3 for the penetration of the analysed substance is made in the form of a solid fluorographite matrix 4, interplanar spaces thereof acting as a vessel for the analysed substance, and a case 5 wherein said matrix 4 is installed.

In the described embodiment of the apparatus the solid fluorographite matrix 4 is made in the form of a powder 6 located in the case 5 comprising a bushing with fine mesh grids 7 and 8 installed on both faces thereof to provide more uniform distribution of the carrier gas through the means 3.

The housing 1 comprises two parts 10 and 11 connected to each other by means of a threaded connection 12. In the part 10 of the casing 1 there is provided a hole 13 for the carrier gas, and in the part 11 of the casing 1 - an outlet hole 14 for the carrier gas.

If the case 5 is installed in the housing 1 on the side where the carrier gas enters, a gas distributing plate 15 with holes 16 located along its perimeter is mounted in the case 5, and on the side where the carrier gas has its outlet from the case 5 a perforated cover 17 is installed. The air-tightness of installation of the case 5 into the housing 1 is attained with an aid of fluoroplastic gaskets 18.

Said embodiment of the apparatus carrying out the method according to the invention is to be used preferably for analysed substance possessing relatively low values of saturated vapour pressure at a given temperature. In those cases the provision of the fluorographite matrix 4 in the form of the powder 6 ensures the highly developed surface of the matrix 4 blown by the carrier gas, and facilitates the diffusion of the analysed substance from the body of the matrix 4 into the free space which is necessary to attain high concentrations of the analysed substance in the stream of the carrier gas.

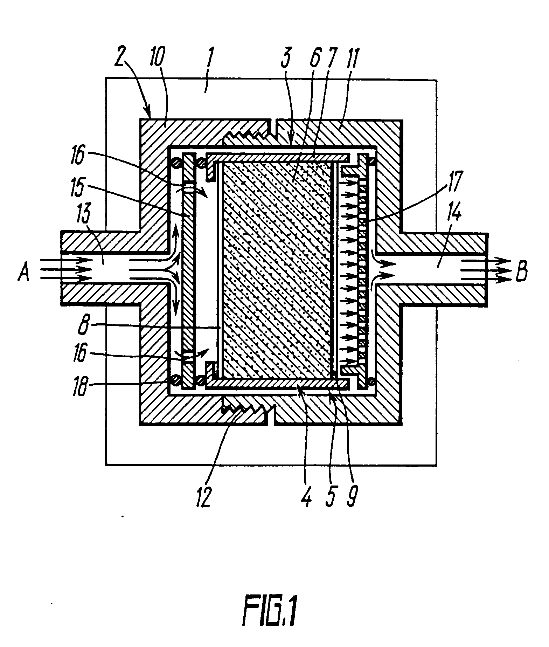

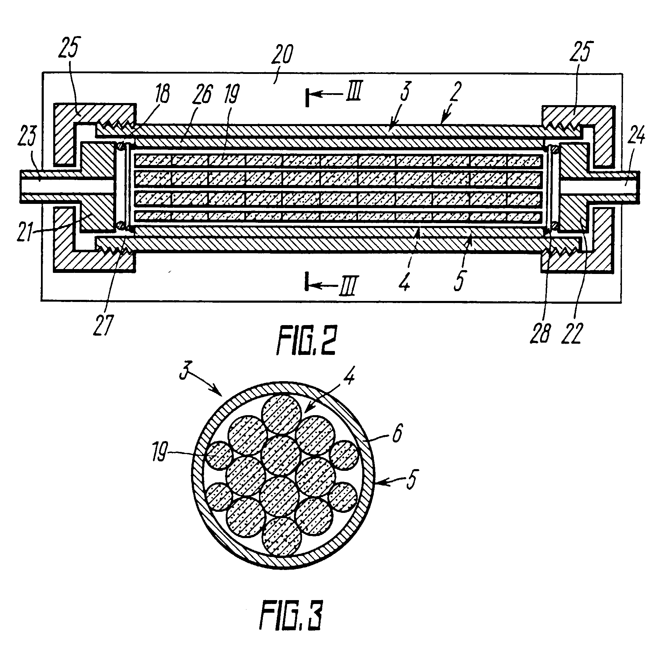

The embodiment of the claimed apparatus for carrying out the method according to the invention represented in Fig.2 is made similar to the apparatus in Fig.1.

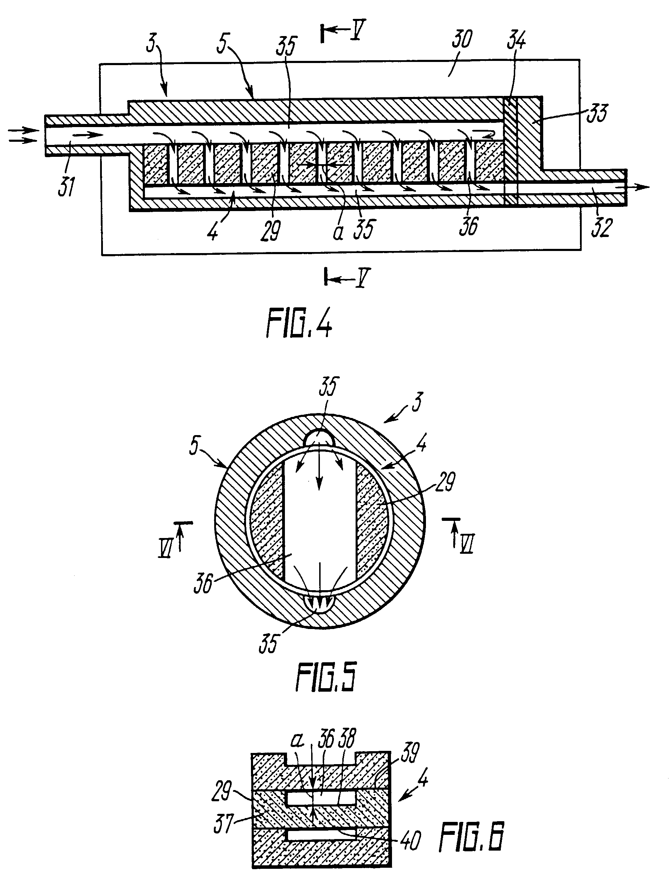

The difference resides in that the solid fluorographite matrix 4 (Figs 2 and 3) is made in the form of assemblage of cylindrical pellets 19 arranged in several rows inside the case 5 along its longitudinal axis at a given distance from one another.

For this purpose the housing 2 (Fig.2) positioned into a thermostatting device 20 has connection points 2 and 22 with an input 23 and an output 24 holes for the carrier gas, said connection points are fastened to the housing 2 by means of nuts 25, and the case 5 consists of a hollow cylinder 26 on facial surfaces whereof fine mesh grids 27 and 28 acting also as fixing members for the pellets 19 are installed.

Said embodiment of the apparatus carrying out the method according to the invention is convenient as applied for the preparation of standard vapour-gaseous mixtures of analysed substances with high saturated vapour pressure and gaseous substances.

The embodiment of the apparatus represented in Fig.4 is made similar to the apparatus represented in Fig.2.

The difference resides in that pellets 29 (Fig.4) of the solid fluorographite matrix are arranged in the space of the case 5 made in the form of a cylinder, mounted directly into a thermostatting device 30 and having an input hole 31 for the carrier gas and an output hole 32 for the carrier gas made in a connection point 33 mounted on the side of one of facial surfaces of the case 5 and acting also as a cover for the case 5. Between the connection point 33 and the face of the case 5 a fluoroplastic gasket 34 is provided wherein a hole is made serving as a continuation of the hole 32.

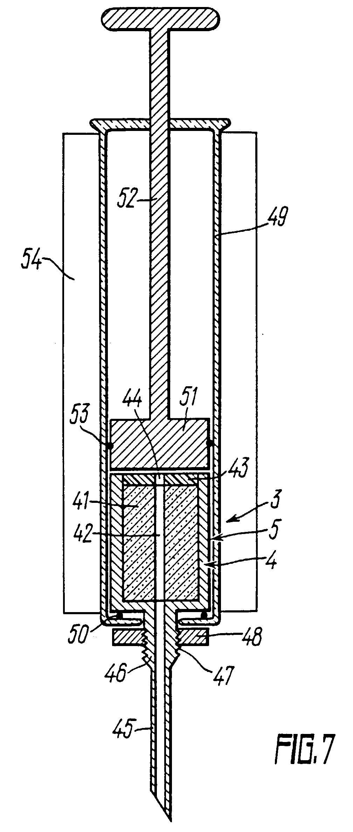

In the case 5 (Figs.4 and 5) passages 35 are made to provide the passage of the carrier gas and distribution of its stream uniformly through passages 36 formed by the pellets 29.

The given distance a (Fig.6) between the pellets 29 of the matrix 4 is determined by the shape of the pellets. For this purpose each pellet 29 is made in the form of a disc 37 having a hollow 38 and a flange 39 along the periphery on one side, and another side 40 thereof is free. The flange 39 of the previous pellet 29 in a row contacts with the free side 40 of each subsequent pellet 29 thus providing the specified distance a.

Said embodiment of the apparatus can be applied for all kinds of substances to be analysed.

The embodiments of the claimed apparatus described hereinabove are intended to prepare standard vapour-gaseous mixtures of substances analysed in continious stram of the carrier gas with an aid of dynamic type gas analysers.

The following embodiments of the apparatus are intended for static type gas analysers when the analysis of substances or vapour-gaseous mixtures is carried out periodically.

In Fig.7 there is represented one more embodiment of the apparatus for the preparation of standard vapour-gaseous mixtures, according to the invention, using static method.

The apparatus in Fig.7 is made similar to the apparatus in Figs.1;2,3;4,5,6.

The difference resides in that the solid fluorographite matrix 4 (Fig.7) is made in the form of a single pellet 41 pressed in the case 5. A passage 42 is made in the pellet 41.

The case 5 is made in the form of a hollow cylinder. On one side of the case 5 cylinder there is located a fluoroplastic gasket 43 provided with an inlet hole 44 for the carrier gas located coaxially with the passage 42. On the other face of the case 5 cylinder there is provided a hollow needle 45 located coaxially with the passage 42 and acting as an outlet hole for the carrier gas. On the outer surface of a needle 45 flange 46 located directly near the face of the case 5 cylinder there is made a thread 47 with a nut 48 placed thereon.

The case 5 is installed in a housing 49 on a fluoroplastic gasket 50 installed on the bottom of the housing 49 so that the needle 45 with the thread 47 and the nut 48 is located outside the housing 49, and the gasket 43 is directed towards a piston 51 with a rod 52, said piston is installed into the housing 49 with the possibility of reciprocating displacement inside the housing 49. The piston is sealed with a fluoroplastic gasket 53.

The housing itself is installed in a thermostatting device 54.

Said embodiment of the claimed apparatus carrying out the method according to the invention is convenient in application for gasochromtographic analysis of substances characterized by high volatility including gaseous substances.

Hereinabove there was described the embodiment of the apparatus wherein a single passage 42 is made in the pellet 41 of the matrix 4. However several passages can be made in the pellet successfully.

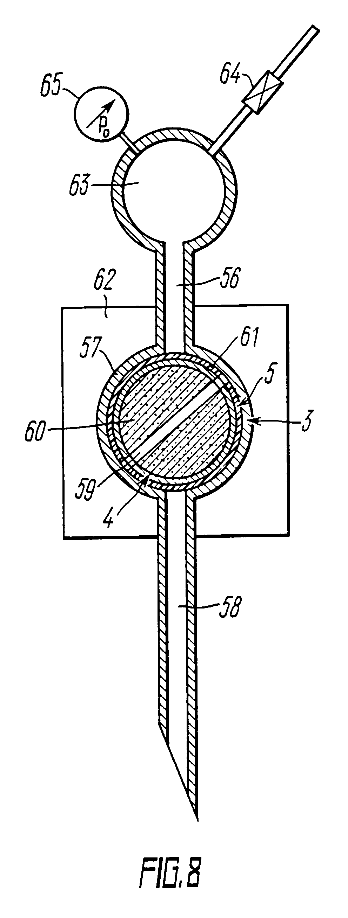

The embodiment of the claimed apparatus according to Figs. 8-10 carrying out the method according to the invention is made similar to the apparatus in Fig.7.

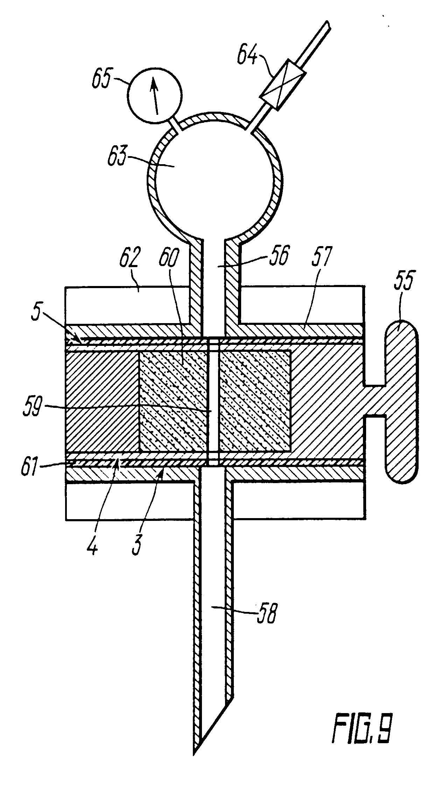

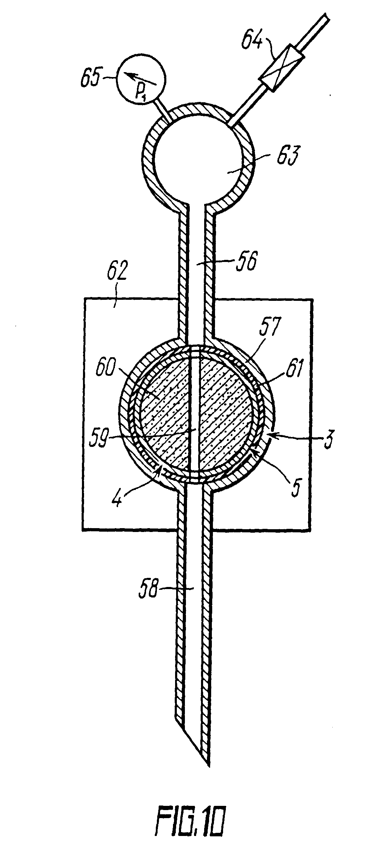

The difference resides in that the case 5 (Fig.8) comprising the solid fluorographite matrix 4 is installed with the possibility of rotation by means of a handle 55 (Fig.9) so that the case 5 either air-tightly shuts off an inlet hole 56 in a housing 57 for the carrier gas from an outlet hole in the housing 57 for the carrier gas, said hole having a form of a hollow needle 58, as shown in Fig.8, or matches a passage 59 provided in a pellet 60 of the matrix 4 with the hole 56 and the needle 58, as it is shown in Fig.10.

The case 5 (Figs.8-10) is made in the form of a solid cylindrical body with the pellet 60 mounted thereinto, and rotates supported by a fluoroplastic gasket 61.

The housing 57 is installed in a thermostatting device 62 and its inlet hole 56 is coupled to a vessel 63 for the compressed carrier gas of a specified volume, said vessel is coupled via a valve 64 to a sourse of the compressed carrier gas (not shown in the drawing) and has a manometer 65.

The present embodiment of the apparatus can be applied for the preparation of standard vapour-gaseous mixtures of all kinds of substances to be analysed.

The operating principle of the claimed apparatus for the preparation of standard vapour-gaseous mixtures carrying out the method according to the invention is as follows.

The estabilished stream of the carrier gas directed as indicated by arrows A (Fig.1) enters the inlet hole 13 of the housing 2 thermostatted by the device 1, in said housing the means 3 for the penetration of the analysed substance is located. Passing over the gas distributing plate 15 the carrier gas through its holes 16 and fine mesh grid 8 uniformly enters the free space of the case 5 wherein the solid fluorographite matrix 4 in the form of the powder 6 is placed.

Molecules of the analysed substance,present in interplanar spaces of the fluorographite matrix 4,by way of diffusion along interplanar spaces leave the body of the matrix 4 and appear on the surface of the powder 6 particles, forming thereon a surface layer of the analysed substance. The diffusion rate depends on the temperature of the thermostatting device 1 and increases as the temperature increases. Further, molecules of the substance are desorbed from the surface layer into the free space of the means 3, said space is blown-through by the carrier gas, wherefrom they are washed out by the stream of said carrier gas; besides, the desorption rate is also determined by the temperature.

The stream of the carrier gas containing the additive of the analysed substance molecules formes the vapour-gaseous mixture and leaves through the fine mesh grid 9, the perforated cover 17 and the outlet hole 14, in the direction indicated by arrows B, into a gas offtake path towards the gas analyser (not shown in the drawing as not being subjects of the present invention).

The operating principle of the apparatus in Figs.2 and 3 is similar to the operating principle of the apparatus in Fig.1.

The difference resides in that the stream of the carrier gas entering the means 3 (Figs.2 and 3) for the penetration of the analysed substance passes only over the surface of the pellets 19 of the fluorographite matrix 4 and cannot remove the analysed substance from the internal space of the pellets 19.

Molecules of the analysed substance, in turn, can participate in generation of vapour-gaseous mixture only after they have diffused from the interplanar spaces of the fluorographite matrix 4 into the internal space of the pellets 19, and from the internal space of the pellets 19 to their surfaces.

In said embodiment of the invention due to reduction of the diffusion rate of molecules into the unconfined space of the means 3 extremely low concentrations of analysed substances in a stream of the carrier gas can be generated which is of especial importance for highly volatile and gaseous substances.

The operating principle of the apparatus in Figs.4-6 is similar to the operating principle of the apparatus in Figs.2 and 3.

The difference resides in that as a result of variation of the pellets' 29 shape (Fig.6) and their location in the case 5 (Figs.4 and 5) the distribution of the carrier gas flow in the free space takes place along curved trajectories which results in that each pellet 29 is passed over by clear carrier gas, and the final flow of the vapour-gaseous mixture is formed in the passages 35 as a sum of smaller flows.

Said apparatus provides more uniform saturation of the carrier gas with molecules of the analysed substance desorbing from the surface of the pellets 29 as compared with the previously described embodiment of the apparatus in Figs.2 and 3 which results in reduction of time required to establish the state of equilibrium and time required for the apparatus to go into steady operating mode.

The operating principle of the apparatus in Fig.7 is similar to the operating principle of the apparatus in Figs.1;2,3; 4,5,6.

The difference resides in that in said apparatus the stream of the carrier gas is not used, and standard vapour-gaseous mixture is prepared in stationary conditions.

A piston 51 (Fig.7) is pushed down as far as it will go and in this state the apparatus according to the invention is heated at the specified temperature for the time required to attain the state of equilibrium. The inlet of the vapour-gaseous mixture having generated in the passage 42 as a result of diffusion of molecules of the analysed substance from the body of the matrix 4 to the walls of the passage 42 and their further desorbtion in the space of the passage 42 is carried out by reciprocating movement of the piston 51 while the volume of the carrier gas pumped through the needle 45 is fixed.

The operating principle of the apparatus in Figs.8-10 is similar to the operating principle of the apparatus in Fig.7.

The difference resides in that the preparation of standard vapour-gaseous mixture takes place into an air-tightly sealed passage 59 (Fig.8), and the inlet of the vapour-gaseous mixture into the gas analyser is carried out pneumatically.

When the apparatus according to the invention starts operating the inlet hole 56 and the hollow needle 58 are shut off by the case 5 as it is shown in Fig.8, and in this state the apparatus is thermostatted for the time required to attain the state of equilibrium.

The introduction of the vapour-gaseous mixture having generated into the passage 59 as a result of diffusion of molecules of the substance from the body of the matrix 4 is carried out pneumatically. For this purpose an excessive pressure Po of the carrier gas is provided into the vessel 63, and then the vessel 63 is air tightly closed by a valve 64. The value of Po is measured by a manometer 65. After the state of equilibrium has been attained the passage 59 by way of the case 5 rotation is matched respectovely with the inlet hole 56 and the hollow needle 58, then the excessive pressure of the carrier gas is released from the vessel 63 to the gas analyser through the passage 59 and the needle 58 as it is shown in Fig.10.

The manometer 65 indicates the reduction of pressure Po down to the value of P1, the difference between said pressures characterizes the volume of the inletted vapour-gaseous mixture.

After the introduction the claimed apparatus returns again to its initial state as it is shown in Fig.8, there is carried out the accumulation of vaporous phase of the analysed substance in the air-tightly sealed passage 59, and the establishment of equilibrium state.

In the state of equilibrium the vapour-gaseous mixture can be kept in the passage 59 as long as it is necessary till the next analysis.

Given below are specific examples of preparation of standard vapour-gaseous mixtures of the analysed substance using the claimed method and the apparatus for carrying out said method, according to the invention.

### Example 1

The apparatus according to Fig.1 is used to generate standard vapour-gaseous mixture.

In the case 5 (Fig.1) the solid fluorographite matrix 4 is located; in interplanar spaces of said matrix the analysed substance, nitrogen dioxide (NO₂) is placed in a quantity of 21 mass % of the mass of the fluorographite matrix 4. The total mass of the fluorographite matrix 4 with NO₂ is 6g.

The apparatus is thermostatted at 40°C. The stabilized stream of the carrier gas is supplied through the inlet hole 13. Nitrogen is used as the carrier gas. Before entering the inlet hole 13 the carrier gas is heated to 40°C by the thermostatting device 1. The flow rate of the carrier gas stream is 14 ml/min. Passing through the apparatus in Fig.1 the carrier gas is enriched by nitrogen oxides and enters the inlet of a chemoluminescent gas analyser-comparator.

For two month of continuous operation of the apparatus according to the invention, results were obtained showing that the level of vapour-gaseous mixture within said time at given flow rate of the carrier gas and temperature of the thermostatting device 1 was sustained with the accuracy of ±1.5%, and the concentration of NO₂ at the outlet of the apparatus was 11 mg/m³.

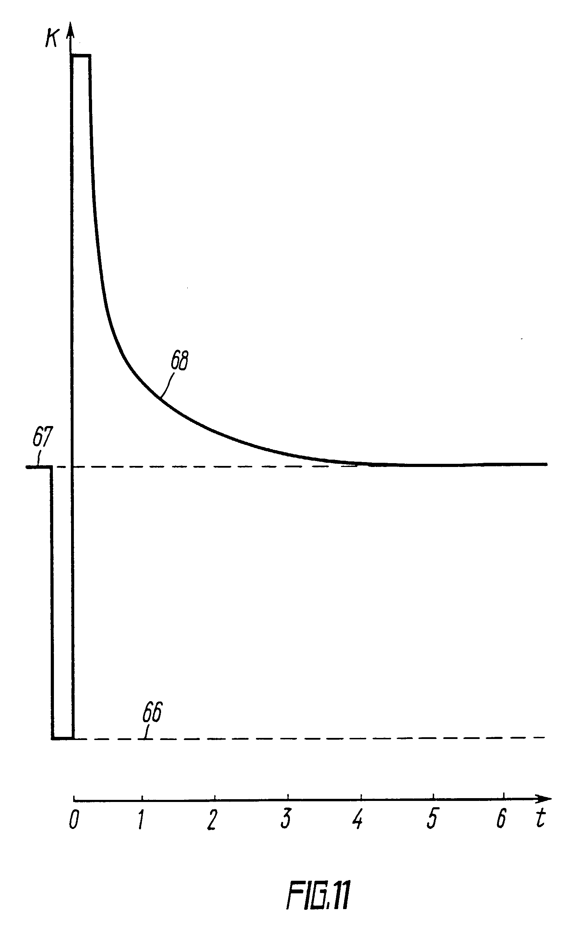

In Fig.11 there is represented a plot of the level of vapour-gaseous mixture of NO₂ in nitrogen registered by the chemoluminiscent gas analyser, wherein time in hours (t) is laid off as abscissa, and the concentration (K) of NO₂ in the stream in mg/m³ is laid off as ordinate.

A level 66 corresponds to the supply of clear carrier gas to the inlet of the gas analyser, a level 67 corresponds to the level of vapour-gaseous mixture of NO₂ generated by the apparatus according to the invention for the period of two months, and a level 68 characterizes the variation of vapour-gaseous mixture which takes place from the moment the apparatus starts its operation till the moment the apparatus attains steady operating conditions.

### Example 2

The apparatus according to Fig.8-10 carrying out the method according to the invention is used to prepare standard vapour-gaseous mixture of the analysed substance - carbon tetrachloride (CCl₄)

The apparatus described hereinabove, in the case 5 whereof the pellet 60 of the fluorographite matrix 4 is located containing 19.6 mass % of CCl₄ in a quantity of 200 mg (diameter of the passage 59 is 0.2 mm, length is 5 mm), is kept at a temperature of the thermostatting device 62 of 50°C for half a year.

Samples of vapour-gaseous mixtures are taken using gas-liquid chromatograph.

For the period of half a year the content of CCl₄ in a vaporous phase, determined by the square of a chromatographic peak, remained stable and representable within the limits of determination error ±5%.

The time required for the claimed apparatus to go into steady operating conditions (from the beginning of heating till the establishment of constant concentration of CCl₄ vapours in the passage 59) was 7 days. The minimal time required to establish the equilibrium state for each analysis is 5 min, Longer intervals between analyses (from 5 min to several days) do not influence the concentration of CCl ₄ in gaseous phase; absolute content of CCl₄ in the analysed mixture was 5·10⁻⁸ g.

Within said period the present apparatus was used to carry out more than 3000 analyses of vaporous phase of the analysed substance, and the concentration of CCl₄ did not change.

The application of the apparatus carrying out the method according to the invention in the analysis of vaporous phases, and as a metrological means for the preparation of standard vapour-gaseous mixtures allows to increase considerably the efficiency of analysis and to simplify the procedure of calibration of a gasoanalytical instrument. This is the result of combination in a single apparatus of functions of preparation of vapour-gaseous mixture, its sampling, measuring, storage and introduction in a gas analyser; the reduction to minimum of the "equipment memory" effect thanks to exclusion of contact of components of vaporous phase with the walls of the passage made in a pellet, which provides the possibility of multiple repetitions of successive measurings in analytical purposes; the reduction of extreme values of concentrations of the analysed substances in vapour-gaseous mixtures, and the increase of accuracy of preparation of said mixtures.

Moreover, said apparatus enables to reduce considerably the overall dimensions of a construction and reduce energy consumption for its thermostatting.

Thus, the claimed method carried out in the apparatus according to the invention provides the possibility of generation of vapour-gaseous mixtures of the analysed substance by means of its diffusion into a free space from a thermostatted fluorographite matrix with the analysed substance located in interplanar spaces thereof.

The claimed method is carried out in two embodiments of the apparatus allowing to generate standard vapour-gaseous mixtures of the analysed substance in a free space both in static and dynamic modes of operation in a stream of a carrier gas. The transition from one level of concentrations of the analysed substance vapour-gaseous mixtures to the other one can be carried out in the same apparatus by varying the temperature of thermostatting.

The apparatus carrying out the claimed method for the preparation of standard vapour-gaseous mixtures of the analysed substance has low inertia thanks to short diffusional path of the analysed substance, allows to generate standard vapour-gaseous mixtures of analysed substances independently on their states of aggregation and chemical properties, possesses long sevice life, is ecologically safe in production, storage, transportation and operation.

High capacity of the solid fluorographite matrix for the analysed substance (up to 20 mass %) enables to carry out the claimed method in a compact apparatus, integrate it into measuring and technological systems. Low inertia of the present apparatus enables to automatize the calibration of gas analysers using the generated standard vapour-gaseous mixtures of the analysed substance.

### Industrial Applicability

The claimed method carried out by the apparatus according to the invention can be used as a metrological support of gas analysing equipment in metrology, medicine, environment protection, analytical chemistry and microelectronics.

## Claims

1. A method for the preparation of standard vapour-gaseous mixtures of a substance to be analysed, the method comprising thermostatting of the substance and diffusion of the substance into a free space through which a carrier gas flows,
characterised in that the diffusion of the substance into the free space through which the carrier gas flows is carried out during thermostatting, at a temperature of up to 50°C, from a solid fluorographite matrix which is chemically inert to the substance to be analysed, the substance being located in interplanar spaces of the matrix and escaping from the matrix by diffusion.

2. Apparatus for the preparation of standard vapour-gaseous mixtures of a substance to be analysed, comprising a thermostatting device (1) which accommodates an enclosure (2) defining a free space through which a carrier gas is to flow, the enclosure (2) containing the substance to be analysed,
characterised in that the enclosure (2) contains a solid fluorographite matrix (4) which is chemically inert to the substance to be analysed, the substance being located in interplanar spaces of the matrix and escaping from the matrix by diffusion at a thermostatting temperature of up to 50°C.

3. Apparatus as claimed in claim 2, in which the solid fluorographite matrix (4) is in the form of a powder (6).

4. Apparatus as claimed in claim 2, in which the solid fluorographite matrix (4) is in the form of at least one pellet (29;41;60).

5. Apparatus as claimed in claim 4, in which a plurality of pellets (29) are arranged at a preset distance (a) from one another.

6. Apparatus as claimed in claim 4, in which the pellet (41;60) has at least one passage (42;59).

7. Apparatus as claimed in claim 6, in which the pellet (60) is rotatable.

## Patentansprüche

1. Verfahren zur Herstellung von Dampf-Gas-Standardgemischen einer zu analysierenden Substanz, wobei das Verfahren eine Thermostatierung der Substanz und eine Diffusion der Substanz in e-nen freien Raum aufweist, durch den ein Trägergas strömt, dadurch gekennzeichnet, daß die Diffusion der Substanz in den von dem Trägergas durchströmten freien Raum während des Thermostatierens bei einer Temperatur von bis zu 50°C aus einer festen Fluorgraphitmatrix erfolgt, die gegenüber der zu analysierenden Substanz chemisch inert ist, wobei sich die Substanz in den Räumen zwischen den Gitterebenen der Matrix befindet und durch Diffusion aus der Matrix entweicht.

2. Vorrichtung zur Herstellung von Dampf-Gas-Standardgemischen einer zu analysierenden Substanz, mit einer Thermostatiereinrichtung (1), die ein Gehäuse (2) aufweist, das einen freien Raum abgrenzt. durch den ein Trägergas fließen soll, wobei das Gehäuse (2) die zu analysierende Substanz enthält, dadurch gekennzeichnet, daß das Gehäuse (2) eine feste Fluorgraphitmatrix (4) enthält. die gegenüber der zu analysierenden Substanz chemisch inert ist, wobei sich die Substanz in Räumen zwischen den Gitterebenen der Matrix befindet und durch Diffusion bei einer Thermostatierungstemperatur von bis zu 50°C aus der Matrix entweicht.

3. Vorrichtung nach Anspruch 2, wobei die feste Fluorgraphitmatrix (4) in Form eines Pulvers (6) vorliegt.

4. Vorrichtung nach Anspruch 2, wobei die feste Fluorgraphitmatrix (4) in Form mindestens eines Pellets (29; 41; 60) vorliegt.

5. Vorrichtung nach Anspruch 4, wobei mehrere Pellets (29) in einem vorgegebenen Abstand (a) voneinander angeordnet sind.

6. Vorrichtung nach Anspruch 4, wobei das Pellet (41; 60) mindestens einen Durchflußkanal (42; 59) aufweist.

7. Vorrichtung nach Anspruch 6, wobei das Pellet (60) drehbar ist.

## Revendications

1. Procédé de préparation de mélanges standard de vapeur et de gaz d'une substance à analyser, le procédé comprenant la thermostatisation de la substance et la diffusion de la substance dans un espace libre, à travers lequel passe un gaz porteur, caractérisé en ce que la diffusion de la substance dans l'espace libre, à travers lequel passe le gaz porteur, est effectuée pendant la thermostatisation à une température de 50°C au maximum, depuis une matrice massive de fluorographite qui est chimiquement inerte vis à vis de la substance à analyser, la substance étant située dans les espaces interplanaires de la matrice et s'échappant de la matrice par diffusion.

2. Appareil pour la préparation de mélanges standard de gaz et de vapeur d'une substance à analyser, comprenant un dispositif de thermostatisation (1) qui comporte une enceinte (2) définissant un espace libre à travers lequel doit passer un gaz porteur, l'enceinte (2) contenant la substance à analyser, caractérisé en ce que l'enceinte (2) contient une matrice massive de fluorographite (4) qui est chimiquement inerte vis à vis de la substance à analyser, la substance étant située dans les espaces interplanaires de la matrice et s'échappant de la matrice par diffusion à une température thermostatisée de 50°C au maximum.

3. Appareil selon la revendication 2, dans lequel la matrice massive de fluorographite (4) est sous la forme d'une poudre (6).

4. Appareil selon la revendication 2, dans lequel la matrice massive de fluorographite (4) est sous la forme d'au moins une tablette (29; 41; 60).

5. Appareil selon la revendication 4, dans lequel plusieurs tablettes (29) sont disposées à une distance prédéterminée (a) l'une par rapport à l'autre.

6. Appareil selon la revendication 4, dans lequel la tablette (41; 60) a au moins un passage (42; 59).

7. Appareil selon la revendication 6, dans lequel la tablette (60) peut tourner.
